(19) Europäisches Patentamt
European Patent Office
Office européen des brevets

(11) Publication number: 0 444 778 A1

# (12) EUROPEAN PATENT APPLICATION

(21) Application number: 91300608.6

(51) Int. Cl.$^5$: A61K 47/26

(22) Date of filing: 28.01.91

(30) Priority: 14.02.90 US 480471

(43) Date of publication of application:
04.09.91 Bulletin 91/36

(84) Designated Contracting States:
AT BE CH DE DK ES FR GB GR IT LI LU NL SE

(71) Applicant: ALCON LABORATORIES INC
6201 South Freeway
Ft. Worth, TX 76134 (US)

(72) Inventor: Ke, Tai-Lee
2625 Steppington Street
Grand Prairie, Texas 75052 (US)
Inventor: Shaw, Jack Michael
Route 8, Box 408
Mechanicsville, Virgina 23111 (US)

(74) Representative: Kyle, Diana et al
ELKINGTON AND FIFE Beacon House 113 Kingsway
London WC2B 6PP (GB)

## (54) Use of alkyl saccharides to enhance the penetration of drugs.

(57) The use of alkyl saccharides to enhance the penetration of drugs across mucus covered epithelial tissues is disclosed, including enhanced penetrations of topically applied ophthalmic drugs through the corneal epithelium.

EP 0 444 778 A1

# USE OF ALKYL SACCHARIDES TO ENHANCE THE PENETRATION OF DRUGS

The present invention relates to the field of drug delivery across mucus covered epithelial tissues. Such tissues include nasal, pulmonary, rectal, buccal, vaginal, uteral, and gastrointestinal routes for drug administration. More particularly, this invention relates to enhancement of the penetration of ophthalmic drugs and other therapeutic agents through the cornea and other tissues of the eye such as the sclera and conjunctiva.

In order for an ophthalmic drug to be therapeutically effective, it is generally necessary for the drug to penetrate the cornea and be taken up in the aqueous humor, ciliary processes and other tissues in the eye. There are notable exceptions to this general rule, such as drugs or drug products which produce a therapeutic effect by acting on the exterior surface of the cornea (e.g., drugs or drug products useful in improving ocular comfort and/or treating dry or irritated eyes). However, the treatment of conditions involving physiological mechanisms within the eye (e.g., glaucoma, diabetic retinopathy, cataracts, etc.) generally does require the movement of topically applied ophthalmic drugs through the cornea.

In order for a drug to pass through the cornea, it must penetrate three layers of tissue, namely, the epithelium, stroma and the endothelium. Except for highly lipophilic drugs, the epithelium is the main barrier to drug penetration of the cornea. Penetration of the stroma basically involves diffusion of the drug through a barrier which is approximately 360 microns thick. There are currently no known methods of enhancing drug penetration through the stroma or endothelium. However, it is possible to enhance the penetration of drugs through the epithelium, and thereby enhance the overall absorption of drugs applied topically to the eye. The present invention is directed to such enhancement.

There have been prior attempts to enhance the penetration of drugs through the corneal epithelium. The goal of such attempts has generally been to enhance penetration of drugs through the corneal epithelium to an optimal point at which the stroma alone controls drug transport through the cornea. The prior attempts have included use of chemical agents to enhance the penetration of drugs through the epithelium. It has been reported that benzalkonium chloride (BAC), bile salts, dimethyl sulfoxide (DMSO), ethylenediamine tetraacetate (EDTA) and 1-dodecylazayl-cycloheptan-2-one (AZONE$^R$) enhance the corneal penetration of certain drugs. The following publications may be referred to for further background concerning the use of such agents to enhance corneal penetration: Acta Ophthalmologica, Vol.53, p.335 (1975); J. Pharm. Pharmacol., Vol.39, p.124 (1987); Chem. Abstracts, Vol.106, 125931t, p.402 (1987); Journal of Pharmaceutical Sciences., Vol.77, No.1 (Jan.,1988); and Investigative Ophthalmology and Visual Science, Vol.29, No.2 (Feb.,1988). Notwithstanding such prior attempts, there continues to be a need for a means of safely and effectively enhancing the penetration of drugs through the cornea.

A principal objective of the present invention is to provide for a method of enhancing drug delivery across mucus covered epithelial tissues, particularly those of the cornea, sclera and conjunctiva. A further objective of the present invention is to provide topical ophthalmic compositions containing one or more agents for enhancing the penetration of the active ingredient(s) contained therein.

The foregoing objectives and other general objectives of the present invention are satisfied by the provision of a means of enhancing penetration by using a class of compounds referred to herein as surfactants derived from saccharides, or "alkyl saccharides" to enhance the penetration of ophthalmic drugs through the corneal epithelium, sclera and conjunctiva. In addition, the objectives of the present invention are furthered when viscosity enhancing polymers are used in conjunction with the alkyl saccharides to ensure the alkyl saccharides and ophthalmic drugs are retained in the eye for a relatively longer period of time, thus allowing the enhancers more time to facilitate drug transport through the cornea, sclera and conjunctiva.

The invention is illustrated by the following drawings wherein

Figure I compares the amount of a drug, para-aminoclonidine, found in the aqueous humor of rabbits which were administered, the drug with and without the alkyl saccharide, dodecyl maltoside.

Figure II compares the amount of a drug, para-aminoclonidine, found in the aqueous humor of rabbits which were administered the free drug, or the drug in combination with hydroxypropylmethyl cellulose (HPMC) and dodecyl maltoside, or the drug with HPMC.

The present invention is based on the discovery that surfactants derived from alkyl saccharides effectively and safely enhance the corneal penetration of ophthalmic drugs. When "corneal penetration" is used herein it includes penetration through the cornea, sclera and conjunctiva of the eye. These penetration enhancers can be used in compositions comprising any ophthalmic drug which, to be effective, must be substantially taken up by the aqueous humor, ciliary processes and other tissues in the eye upon topical administration. Examples of classes of ophthalmic drugs with which the alkyl saccharides of the present invention can be used, include: steroids, growth factors, cycloplegics, miotics, mydriatics, therapeutic proteins and peptides, antioxidants, aldose reductase inhibitors, nonsteroidal antiinflammatories, immunomodulators, antiallergics, antimicrobials

angiostatic agents and anti-glaucoma therapeutic agents.

The penetration enhancing alkyl saccharides used in the present invention have the following structure:

$$R_1\text{-}Z\text{-}(R_2)_x$$

wherein $R_1$ is a hydrophobic group including saturated and unsaturated aliphatic hydrocarbon groups which range from 8 to 28 carbons in length with 0 to 5 double bonds. The aliphatic hydrocarbon group can be a straight or branched chain and may be substituted by one or more aromatic, cycloaliphatic or hydrophilic (e.g. hydroxyl, thiol, ester or amino) groups.

$R_2$ is a group derived from any saccharide containing 4 - 7 carbons and their isomers;

X is an integer from 1-10; and

Z is -0-, or a carboxyl, amide, phosphate or sulfide group where $R_2$ is covalently bound to such group.

More specifically $R_1$ can be a straight 10-18 carbon alkyl chain in hemiacetal linkage to the saccharide; and $R_2$ a group derived from any of a variety of isomeric saccharides containing 5 or 6 carbons. The saccharide can be, for example, an aldehyde-containing saccharide (glucose, mannose, arabinose, galactose, xylose); a ketone-containing saccharide (fructose, xylulose, sorbose); a saccharide alcohol (sorbitol, inositol, xylitol, mannitol); a saccharide acid (glucuronic acid, neuramiaic acid, mannuronic acid); a deoxysaccharide (deoxy-ribose, rhamnose, fructose); an aminosaccharide (glucosamine, galactosamine); or a derivatized saccharide (alkyl, alkoxyl, amino acids, thiol). Higher order saccharides being covalently linked in any of a number of ways to form different isomeric structures include for example disaccharides such as maltose, cellobiose, sucrose and lactose and trisaccharides, such as raffinose.

The preferred penetration enhancers are alkyl chain containing surfactants derived from maltose and glucose with $R_1$ being 10 to 18 carbons and having the following structures:

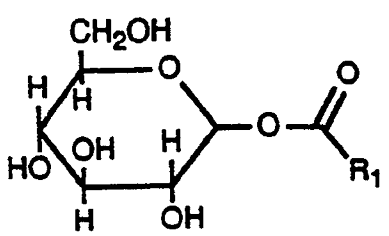

[A]

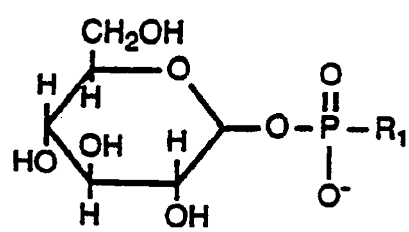

[B]

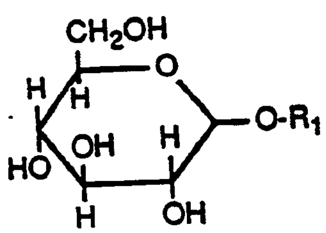

[C]

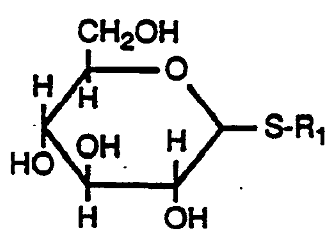

[D]

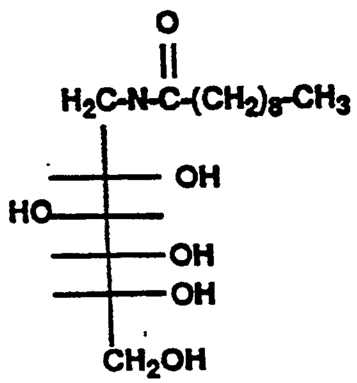

[E]

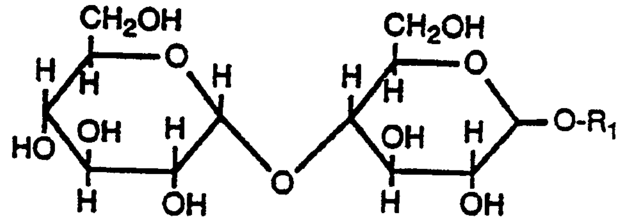

[F]

The most preferred penetration enhancer is:

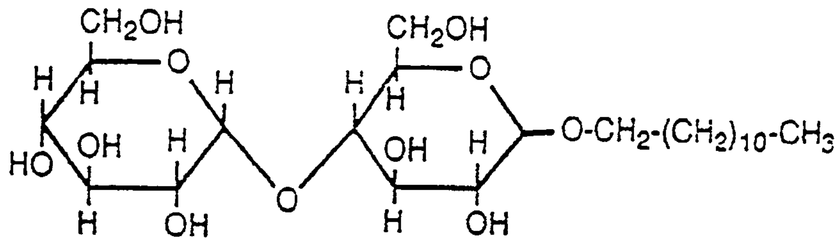

Dodecyl Maltoside

The alkyl saccharides which are useful in the present invention may be described as being "amphipathic", since they include both hydrophilic and hydrophobic groups. While not wishing to be bound by any theory, it is believed that alkyl saccharides enhance the corneal penetration of drugs by partition and interaction with protein, glycoprotein and lipid components present in the membrane of the corneal epithelium. Such interaction is believed to alter the degree of order of the proteins and lipids in the membrane, thereby modifying the function of the epithelium as a barrier to drug penetration. Whatever the mechanism, the net result is that drug penetration of the epithelium is enhanced.

The use of alkyl saccharides in accordance with the present invention to enhance corneal penetration of drugs significantly increases the amount of drug which is able to penetrate the cornea. The degree of enhancement will vary with different drugs, but in some cases may be as much as 3-fold or more. Because drugs can more effectively penetrate the cornea, less drug is lost due to flow down the punctum and therefore less drug need be administered to effectively treat a particular indication. This is particularly beneficial when it is necessary to administer drugs which cause severe systemic side effects.

The amount of alkyl saccharide required in order to enhance corneal penetration will depend on various factors, such as the solubility, partition coefficient and molecular weight of the ophthalmic drug or therapeutic agent; the excipients (surfactants, preservatives, polymers) present in the formulation; and the particular alkyl saccharide being used. In general, the more lipophilic the drug to be delivered, the less alkyl saccharide is required to enhance penetration, and the higher the concentration of alkyl saccharide, the better the corneal penetration. Typically, one or more alkyl saccharides will be used in an amount of from about 0.01% to about 20%(weight/volume) preferably from about 0.05 to 1.0%.

The alkyl saccharide can be used with certain topical drug delivery systems wherein an excipient or vehicle will not substantially impair or prevent the alkyl saccharides from functioning as corneal penetration enhancers. For example, the alkyl saccharide can be formulated in compositions which are solutions, suspensions, ointments, gels or films. The type of composition will depend on, among other things, the chemical and physical properties of the drug or therapeutic agent to be delivered and the properties of polymeric materials used in the formulation. These properties are well known to a person of ordinary skill in the art of drug formulation and delivery.

In a preferred embodiment, the present invention further comprises the use of polymers in conjunction with

the alkyl saccharides to enhance ocular bioavailability. The longer a topical ophthalmic formulation is in contact with the eye the better the ocular bioavailability. Through the use of polymers in conjunction with the above described alkyl saccharides the compositions of the present invention are retained on the cornea longer. As a result, the penetration enhancing components of the compositions can more effectively interact with the corneal epithelium to enhance penetration of the desired drugs or therapeutic agents into the eye. It has been found that the use of polymers in conjunction with alkyl saccharides can provide for up to about a 9 to 10 fold increase in the amount of drug or therapeutic agent made available to the tissues. The effectiveness of the alkyl saccharides can be improved when the viscosity of the compositions containing the alkyl saccharides is increased up to about 1000 centipoise (cps), preferably between about 50 cps. to 300 cps. Polymers are added to provide for this desired viscosity increase.

Any synthetic or natural polymer which will increase viscosity and is compatible with tissues of the eye and the ingredients of the alkyl saccharides compositions can be used. Such polymers are referred to herein as "viscosity enhancing, ophthalmically acceptable polymers." Examples include, but are not limited to: natural polysaccharides and gums, such as alginates, carrageenan, guar, karaya, locust bean, tragacanth and xanthan; and synthetic polymers, such as carbomer, hydroxyethylcellulose (HEC), hydroxypropylcellulose, hydroxypropylmethylcellulose (HPMC), methylcellulose, polyvinyl alcohol (PVA), polyvinyl pyrrolidone carboxymethylcellulose and agarose. In addition, proteins, synthetic polypeptides and polymer-peptide copolymers which enhance viscosity and are ophthalmically acceptable can be used to increase the viscosity of the compositions to provide for better bioavailability. Typically, proteins which can be used include: gelatin, collagen, albumin and casein.

The preferred viscosity enhancing agents are one or more polymers selected from: PVA, HPMC and HEC. The most preferred agent is HPMC. The viscosity enhancing agents are added to provide for compositions with a viscosity of between about 50 and 300 cps.

The preferred method for enhancing the penetration of a drug or therapeutic agent comprises the use of dodecyl maltoside at a concentration of about 0.05% to 1.0% in combination with the polymer, HPMC, in an amount sufficient to provide a composition with a viscosity of about 50 to about 300 cps.

The following examples further illustrate the compositions which, according to the present invention, comprise the corneal penetration enhancing properties of the alkyl saccharides and their use to enhance corneal penetration.

## EXAMPLE 1

The following formulation is an example of a topical ophthalmic composition which can be used to treat glaucoma.

### Formulation

| Ingredients | % (weight/volume) |
|---|---|
| Para-amino-clonidine | 0.125 |
| Dodecyl maltoside | 0.05 |
| Benzalkonium chloride | 0.01 |
| Disodium Edetate, USP | 0.01 |
| Sodium phosphate, monobasic, USP | 0.18 |
| Sodium phosphate, dibasic, USP | 0.12 |
| Mannitol, USP | 3.3 |
| HCl, NF and/or NaOH, NF | q.s. pH to 6.5$\pm$0.2 |
| Purified Water, USP | q.s. 100 |

Procedure for Preparation of Formulation

Approximately 85% (8.5ml) of the batch volume of purified water was added to a container. All of the ingredients were added to the container: 0.018g monobasic sodium phosphate; 0.012g dibasic sodium phosphate; 0.33g mannitol; 0.1ml of 1.0% BAC; 0.001g disodium edetate; 0.0125g para-amino-clonidine. The ingredients were mixed well and stirred until all ingredients dissolved into a solution. 0.005g dodecyl maltoside was added to the container and sonnicated for 5 minutes. The pH was adjusted to pH 6.5. Purified water was then poured through a sterilizing filter into the container (q.s. to 10ml) and the solution was mixed well.

Nine New Zealand albino rabbits were selected for evaluation of the penetration through the cornea of the para-amino-clonidine formulation set forth above. All rabbits received 30ul of the 0.125% para-amino-clonidine topically in both eyes. Three rabbits were sacrificed at 20 minutes from dosing and aqueous humor was withdrawn from their eyes. The aqueous humor was assayed by liquid scintillation counting to determine the amount of para-amino-clonidine in the aqueous humor. The same procedure was done on 3 different rabbits at 60 minutes from dosing and on another 3 rabbits, 120 minutes from dosing. Nine control rabbits received 0.125% para-amino-clonidine as set forth in the formulation above without 0.05% dodecyl maltoside. Aqueous humor was withdrawn and assayed as explained above. The results are shown in the graph depicted in Figure I. It can be seen from the graph that the amount of para-amino-clonidine in the aqueous humor is greater in the rabbits treated with the formulation containing dodecyl maltoside. At 60 minutes there is almost a four fold increase in the amount of para-amino-clonidine found in the aqueous humor of those rabbits which received the drug in conjunction with dodecyl maltoside versus those who received the drug without dodecyl maltoside. Therefore, the results indicate that dodecyl maltoside enhanced penetration of para-amino-clonidine through the cornea.

**EXAMPLE 2**

Formulation

| Ingredients | % (weight/volume) |
|---|---|
| Para-amino-clonidine | 0.125 |
| Hydroxypropylmethylcellulose-E50LV,(HPMC)USP | 3.1 |
| Dodecyl maltoside | 0.05 |
| Benzalkonium chloride | 0.01 |
| Disodium Edetate, USP | 0.01 |
| Sodium phosphate, monobasic, USP | 0.18 |
| Sodium phosphate, dibasic, USP | 0.12 |
| Mannitol, USP | 3.3 |
| HCl, NF and/or NaOH, NF | q.s. pH to 67.5±0.2 |
| Purified Water, USP | q.s. 100 |

Procedure for Preparation of Formulation

Approximately 85% (8.5ml) of the batch volume of purified water was added to a container. All of the ingredients were then added to the container: 0.018g monobasic sodium phosphate; 0.33g mannitol; 0.1ml of 1.0% BAC; 0.001g disodium edetate; 0.0125g para-amino-clonidine; 0.31g HPMC. The ingredients were mixed well. 0.005g dodecyl maltoside was added to the container and sonnicated 5 minutes. The pH was adjusted to pH 6.5. Purified water was then poured through a sterilizing filter into the container (q.s. to 10ml) and the solution was mixed well.

Nine New Zealand albino rabbits were selected for evaluation of the penetration through the cornea of the para-amino-clonidine formulation set forth above. All rabbits received 30ul of the 0.125% para-amino-clonidine topically in both eyes. Three rabbits were sacrificed at 20 minutes from dosing and their aqueous humor was withdrawn from their eyes. The aqueous humor was assayed by liquid scintillation counting to determine the amount of para-amino-clonidine in the aqueous humor. The same procedure was done on three different rabbits at 60 minutes from dosing and on another three rabbits, 120 minutes from dosing. Nine control rabbits received 0.125% para-amino-clonidine as set forth in the formulation above without 0.05% dodecyl maltoside and 3.1% HPMC. Another 9 rabbits received 0.125% para-amino-clonidine as set forth in the formulation above without 0.05% dodecyl maltoside. Aqueous humor was withdrawn and assayed as explained above. The results are shown in the graph depicted in Figure II. It can be seen from the graph that the amount of para-amino-clonidine in the aqueous humor is greater in the rabbits treated with the formulation containing HPMC and HPMC with dodecyl maltoside. At 60 minutes there is almost a 4.0 fold and a 10 fold increase in the amount of para-amino-clonidine found in the aqueous humor of those rabbits which received the drug in conjunction with HPMC or with HPMC and dodecyl maltoside, respectively, as compared to those which received the drug without HPMC or HPMC and dodecyl maltoside. The results indicate that dodecyl maltoside enhances the penetration of para-amino-clonidine through the cornea over HPMC alone and para-amino-clonidine alone.

## Claims

1. A topical, pharmaceutical composition comprising: a therapeutically effective amount of a drug and an amount of an alkyl saccharide effective to enhance penetration of the drug across mucus covered epithelial tissue.

2. The composition of claim 1 wherein the alkyl saccharide has the formula

$$R_1\text{-}Z\text{-}(R_2)_x$$

wherein, $R_1$ is a hydrophobic group including saturated and unsaturated aliphatic hydrocarbon groups which range from 8 to 28 carbons in length with 0 to 5 double bonds and said aliphatic hydrocarbon group can be a straight or branched chain and may be substituted by one or more aromatic, cycloaliphatic or hydrophilic groups;
$R_2$ is a group derived from any saccharide containing 4 - 7 carbons;
X is an integer from 1-10; and
Z is -0-, or a carboxyl, amide, phosphate or sulfide group where $R_2$ is covalently bound to such group.

3. The composition of claim 2 wherein the alkyl saccharide is selected from the group consisting of:

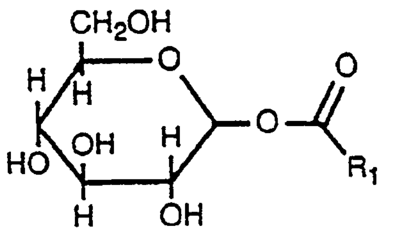

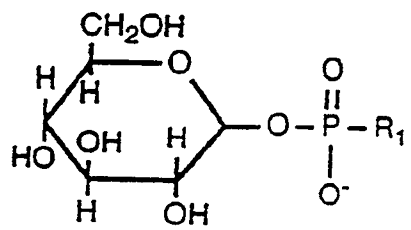

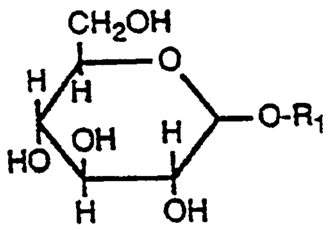

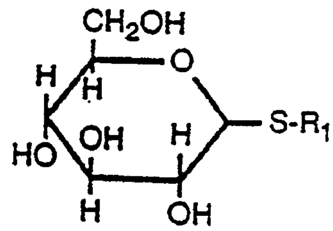

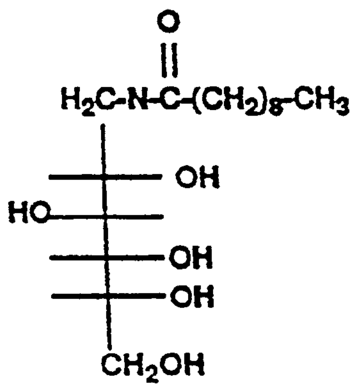

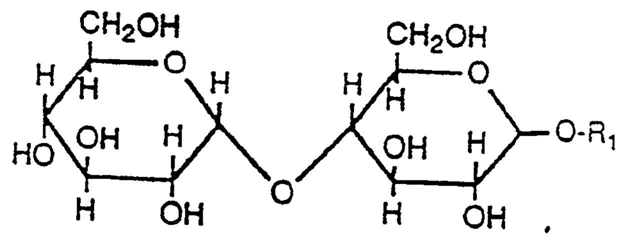

.

4. The composition of claim 2 wherein the alkyl saccharide comprises dodecyl maltoside.

5. The composition of any of claims 1 to 4, wherein the alkyl saccharide concentration is about 0.01 to 20%.

6. The composition of claim 5 wherein the concentration is about 0.05 to 1.0%.

7. The composition of any of claims 1 to 6, which further comprises a polymer in an amount sufficient to provide the composition with a viscosity of up to about 1,000 centipoise.

8. The composition of claim 7, wherein the composition comprises a polymer selected from the group consisting of: alganates, carrageenan, gar, karaya, locust beans, tragagcanth, zanthan, carbomer, hydroxyethylcellulose, hydroxypropylcellulose, hydroxypropylmethylcellulose, methylcellulose, polyvinyl alcohol, polyvinyl pyrrolidone, carboxymethylcellulose and agarose.

9. The composition of claim 8 wherein the polymer is hydroxypropylmethylcellulose.

10. The composition of claim 9 wherein the alkyl saccharide comprises dodecyl maltoside.

11. The composition of claim 10 wherein the alkyl saccharide concentration is between about 0.05 and 1.0% and the hydroxy propylmethylcellulose is present in an amount sufficient to provide the composition with a viscosity of between about 50-300 cps.

12. Use of a topical pharmaceutical composition to enhance penetration of a drug across mucus-covered epithelial tissue, the pharmaceutical composition comprising: a therapeutically effective amount of a drug and a sufficient amount of an alkyl saccharide to enhance penetration of said drug across the mucus covered epithelial tissue.

13. The use according to claim 12 of a composition as claimed in any of claims 2 to 11.

Figure I
Concentration of para-amino Clonidine in Rabbit Aqueous Humor with and without Dodecyl Maltoside

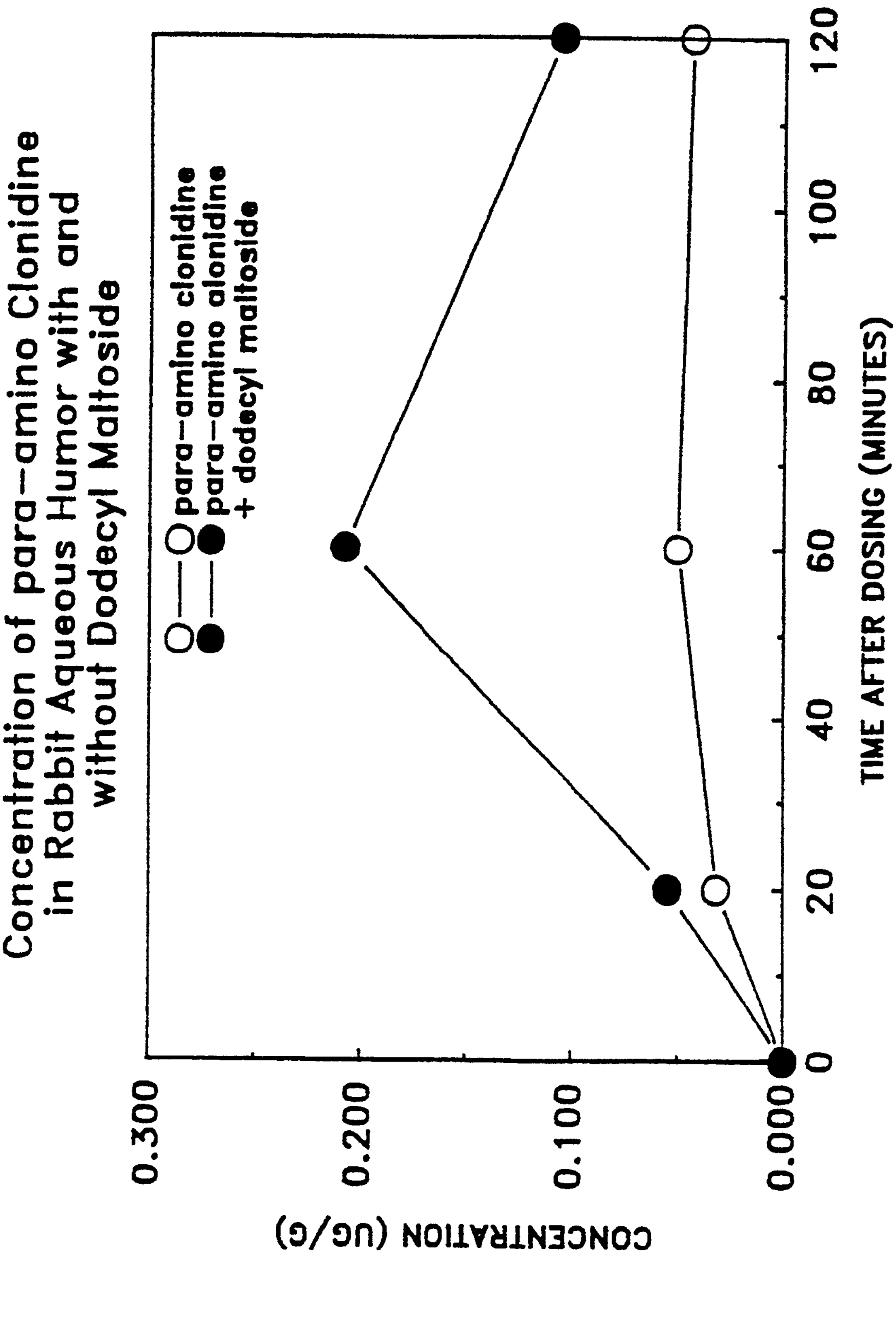

Figure II
Concentration of Para–Amino Clonidine in Rabbit Aqueous Humor without HPMC, with HPMC, and with HPMC and Dodecyl Maltoside

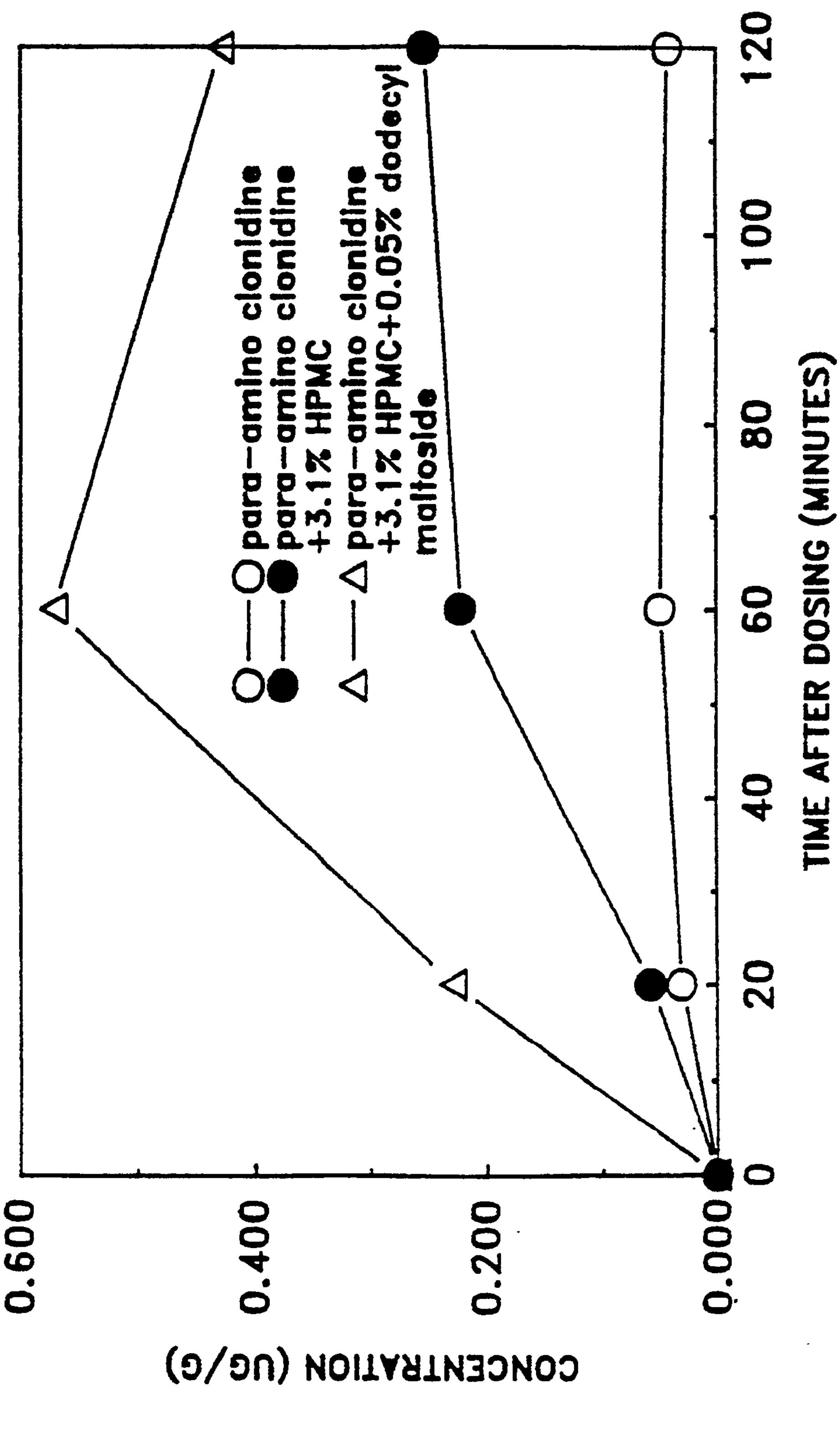

European Patent Office

# EUROPEAN SEARCH REPORT

Application Number

EP 91 30 0608

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN, vol 13, no. 414 (C-635)[3762], 13th September 1989; & JP-A-1 151 528 (TAIHO YAKUHIN KOGYO K.K.) 14-06-89 * Abstract * | 1-6,12, 13 | A 61 K 47/26 |
| Y | IDEM | 7-11 | |
| Y | EP-A-0 183 457 (FISENS plc) * Page 2, line 21 - page 5, line 1; page 10, example 2; claim 1 * | 7-11 | |
| P,X | EP-A-0 396 777 (OTSUKA PHARMACEUTICAL CO., LTD) * Page 2, lines 10-25; page 4, lines 3-13; pages 6,7, example 1; claims * | 1,2,5,6 ,12,13 | |
| X | EP-A-0 280 413 (ALZA CORP.) * Page 2, lines 20-33; page 4, example 1; claims 1-3 * | 1,2,5,7 ,12,13 | |
| A | FR-A-2 182 935 (THE PROCTER AND GAMBLE CO.) * Page 2, line 24 - page 3, line 5; page 6, lines 9-34; pages 13-15, example 3; claims 1-5,8,10 * | 1-7 | TECHNICAL FIELDS SEARCHED (Int. Cl.5)<br>A 61 K |

The present search report has been drawn up for all claims

| Place of search | Date of completion of the search | Examiner |
|---|---|---|
| THE HAGUE | 30-05-1991 | BOULOIS D.J-M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)